(19) Europäisches Patentamt
European Patent Office
Office européen des brevets

(11) Veröffentlichungsnummer: **0 097 936**
**B1**

(12) # EUROPÄISCHE PATENTSCHRIFT

(45) Veröffentlichungstag der Patentschrift: **04.12.85**

(51) Int. Cl.⁴: **C 07 K 7/06**, A 61 K 37/02, A 61 K 39/39

(21) Anmeldenummer: **83106213.8**

(22) Anmeldetag: **25.06.83**

(54) **Nonapeptid mit immunstimulierender Wirkung, Verfahren zu dessen Herstellung und dessen Verwendung.**

(30) Priorität: **30.06.82 DE 3224379**

(43) Veröffentlichungstag der Anmeldung: **11.01.84 Patentblatt 84/2**

(45) Bekanntmachung des Hinweises auf die Patenterteilung: **04.12.85 Patentblatt 85/49**

(84) Benannte Vertragsstaaten: **AT BE CH DE FR GB IT LI LU NL SE**

(56) Entgegenhaltungen: **NATURE, Band 285, Nr. 5766, 19. Juni 1980, Seiten 542-547, Macmillan Journals Ltd., R. DERYNCK et al.: "Isolation and structure of a human fibroblast interferon gene"**

(73) Patentinhaber: **HOECHST AKTIENGESELLSCHAFT, Postfach 80 03 20, D-6230 Frankfurt am Main 80 (DE)**

(72) Erfinder: **König, Wolfgang, Dr., Eppsteiner Strasse 25, D-6238 Hofheim am Taunus (DE)**
Erfinder: **Geiger, Rolf, Prof. Dr., Heinrich-Bleicher-Strasse 33, D-6000 Frankfurt am Main 50 (DE)**
Erfinder: **Obermeier, Rainer, Dr., Langenhainer Weg 14, D-6234 Hattersheim am Main (DE)**
Erfinder: **Müllner, Hubert, Dr., Pestalozzistrasse 4, D-6233 Kelkheim (Taunus) (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

**Beschreibung**

Die Erfindung betrifft ein Nonapeptid der Formel I

Glu-Asp-Ser-Ser-Ser-Thr-Gly-Trp-Asn-OH (I)

(L-Pyroglutamyl-L-aspartyl-L-seryl-L-seryl-L-seryl-L-threonyl-glycyl-L-tryptophyl-L-asparagin) und dessen physiologisch verträgliche Salze mit organischen Basen, Alkali- und Erdalkaliionen, pharmazeutische Präparate sowie ihre Verwendung.

Die Erfindung betrifft außerdem ein Verfahren zur Herstellung dieser Verbindung, dadurch gekennzeichnet, daß das geschützte Peptid der Formel II

Glu-Asp($OBu^t$)-Ser($Bu^t$)-Ser($Bu^t$)-Thr($Bu^t$)-Gly-Trp-Asn-$OBu^t$ (II)

mit in der Peptidchemie bei tryptophanhaltigen Peptiden üblichen Methoden von den Schutzgruppen des tert.-Butyltyps befreit (siehe z. B. R. Geiger und W. König in E. Gross und J. Meienhofer, The Peptides, Academic Press, New York 1981, Seite 82).

Neben der antiviralen Wirksamkeit besitzen Interferone auch eine immunmodulierende Aktivität (Stewart, W. E., The Interferon System, Springer, New York, 1979).

Überraschenderweise weist bereits das Nonapeptid der Formel

Glu-Asp-Ser-Ser-Ser-Thr-Gly-Trp-Asn-OH , (I)

die einer Teilsequenz des human-Fibroblasteninterferons entspricht (Nature 285, 542—547, 1980), in in-vitro-Tests eine starke immunstimulierende Wirkung auf.

Die Synthese des Peptids der Formel II erfolgt mittels Segmentkupplung aus drei Dipeptiden und einem Tripeptid (s. Syntheseschema). Zur Peptidkondensation wird (mit Ausnahme der Synthese von

Glu-Asp($OBu^t$)-OH)

die Dicyclohexylcarbodiimid/1-Hydroxybenzotriazol-Methode (DCC/HOBt) herangezogen.

Glu-Asp($OBu^t$)-OH

wird durch die mit 1-Hydroxybenzotriazol katalysierte Umsetzung von Pyroglutaminsäure-2.4.5-trichlorphenylester

(Glu-OTcp)

mit Asp($OBu^t$) hergestellt. Die Seitenkettenfunktionen und die C-terminale Carboxylgruppe werden als tert.-Butyl-ether ($Bu^t$) bzw. als tert.-Butylester ($OBu^t$) blockiert. Der Benzyloxycarbonylrest (Z), der durch katalytische Hydrierung in Gegenwart von tert.-Butylestern oder tert.-Butylethern selektiv abgespalten werden kann, dient als intermediärer Schutz der Aminofunktionen. Mit Methyl- (OMe) bzw. Ethylestern (OEt) werden die Carboxylgruppen zweier Segmente zwischenzeitlich geschützt. Diese Ester sind stabil bei der katalytischen Hydrierung, lassen sich aber mit Alkali in Anwesenheit von Schutzgruppen des tert.-Butyltyps selektiv abspalten.

Die Schutzgruppen des Peptids der Formel II werden durch Lösen der Substanz in 90prozentiger Trifluoressigsäure abgespalten. Um einer Tertiärbutylierung des Tryptophans entgegenzuwirken, setzt man hierbei zweckmäßig ein Thiol zu. Als besonders vorteilhaft hat sich 1.2-Mercaptoethan erwiesen.

Synthese Schema:

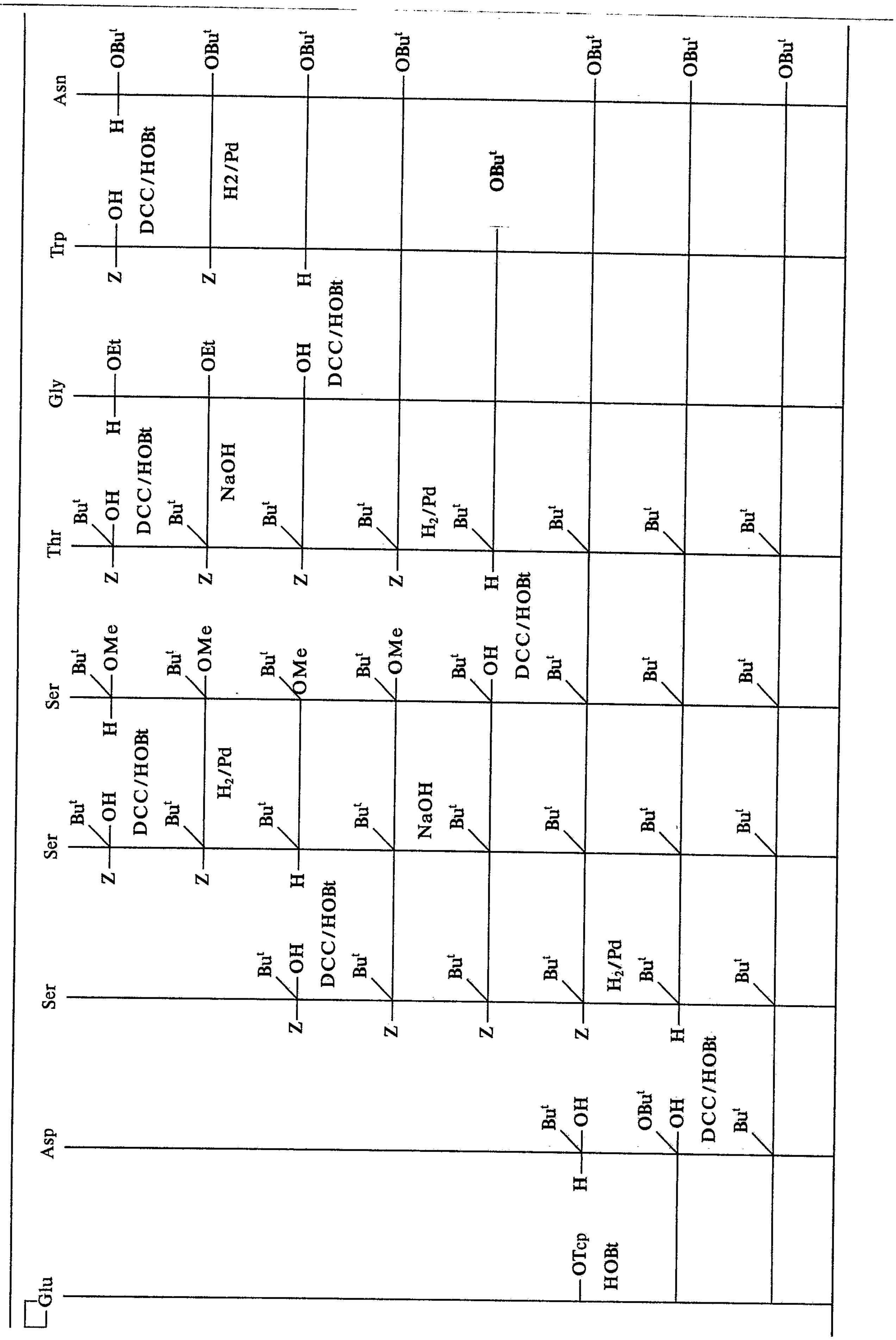

Das erfindungsgemäße Nonapeptid wurde im Plaqueforming Cell Assay (PFC-Test) und im Phythämagglutinin-Stimulationstest (PHA-Test) auf seine lymphozyten-stimulierende Wirkung getestet.

Im PFC-Test werden Zellkulturen aus frischpräparierten Milzen von Mäusen ($2 \times 10^7$ Milzzellen pro ml) in RPMI 1640 und 30 µl foetalem Kälber-Serum (FKS) pro ml Zellkultur angelegt. Die in-vitro Immunisierung erfolgt mit $5 \times 10^7$ Schafserythrozyten/ml. Die Test-Zellkulturen werden täglich mit der entsprechenden Dosis der Testsubstanz inkubiert.

Nach 5tägiger Laufzeit werden die Zellen abzentrifugiert, mit dem Medium RPMI 1640 gewaschen und der direkte PFC-Test durchgeführt. Dazu werden die Zellen zusammen mit einer 10prozentigen Schafserythrozyten-Suspension in einer Agaroselösung gemischt und auf einer ebenen Fläche ausgegossen. In der entstandenen Gelschicht geben die stimulierten Lymphocyten während der anschließenden Inkubation Antikörper ab, die in die Umgebung diffundieren und sich an die hier befindlichen Schafserythrozyten heften. Nach der Zugabe von Meerschweinchen-Komplement lysieren die roten Blutzellen. Es entstehen helle, mit bloßem Auge erkennbare runde Flecken in dem rötlich-braunen Gel, nämlich die Hämolysehöfe der Plaques. Im Zentrum solcher Hämolysehöfe befindet sich eine antikörperproduzierende Zelle. Die Anzahl lymphoider Zellen, die spezifische Immunglobuline bilden, kann folglich mit den ermittelten Plaque-Zahlen gleichgesetzt werden.

Im PHA-Test werden Rückschlüsse über die Anzahl der reifen, d.h. stimulierbaren Lymphocyten durch den Funktionstest auf Stimulierbarkeit mit dem Pflanzenlektin PHA gewonnen. Die Lymphocyten werden durch das Lektin, ähnlich wie durch bakterielle oder virale Antigene zu einer Blastentransformation angeregt. Dieses führt entweder direkt oder durch Ausschüttung von Lymphokinen zu einer Proliferation.

Der Einbau von radio-aktivem Thymidin innerhalb einer bestimmten Zeit ist dann ein Maß für die Anzahl der stimulierten Zellen. Nur die reifen oder immunologisch potenten T-Zellen werden stimuliert. Es läßt sich daher der Einfluß einer Substanz für die Lymphocytenreifung mit diesem Test verfolgen. Allerdings muß die Stimulierung sub-optimal sein, denn bei höheren Konzentrationen werden auch andere Subpopulationen von Lymphocyten stimuliert, und der Effekt läßt sich nicht mehr beobachten. Das erfindungsgemäße Peptid wurde in verschiedenen Konzentrationen zum Kulturmedium gegeben.

In beiden Testsystemen beobachtet man eine dosisabhängige Stimulierung der Lymphocyten durch das erfindungsgemäße Nonapeptid in Form einer Glockenkurve (siehe Tabelle 1 und 2).

Versuchsergebnisse mit dem erfindungsgemäßen Nonapeptid

Tabelle 1

PFC-Test

| | Kontrolle | 1 ng | 10 ng | 100 ng | 1 µg | 5 µg/ml |
|---|---|---|---|---|---|---|
| Plaques/$10^6$ Zellen | 768 | 908 | 1112 | 1024 | 817 | 802 |

Tabelle 2

PHA-Test (Zugabe von 20 µg PHA/ml, Testdauer: 72 Stunden)

| Konzentration des Nonapeptids (µg/ml) | Einbau von $^3$H-Thymidin (cpm) | Stimulationsindex SI |
|---|---|---|
| Kontrolle | 114 311 | |
| 5,0 | 107 670 | 0,94 |
| 1,0 | 113 978 | 1,00 |
| 0,5 | 143 831 | 1,26 |
| 0,125 | 137 519 | 1,20 |
| 0,0125 | 101 594 | 0,89 |

Die erfindungsgemäße Verbindung kann zur Behandlung von Immundefizienzen, viralen und fungoiden sowie chronisch bakteriellen Infekten, Autoimmunkrankheiten sowie zur Therapie von Erkrankungen dienen, die durch Zellen mit immunologisch relevanten Veränderungen der Zellmembranmerkmale (z. B. Tumorzellen) verursacht werden.

Die Erfindung betrifft weiterhin die Verwendung des genannten Peptids ganz allgemein zur Beeinflussung der Reifung von T-Lymphocyten sowie Mittel, die dieses Peptid als Wirkstoff enthält.

Die Anwendung des erfindungsgemäßen Peptids kann intravenös, subcutan oder intranasal erfolgen. Die Dosierung bei parenteraler Gabe liegt bei 0,01—10 mg (ungefähr 0,1—100 µg/kg/Tag), bei intranasaler Gabe 0,1—100 mg je Einzeldosis. Der bei parenteraler Gabe bevorzugte Dosisbereich beträgt 0,1—10, vorzugsweise 0,2—5 µg/kg/Tag. Sie kann in schweren Fällen auch erhöht werden, da toxische Eigenschaften bisher nicht beobachtet wurden. Auch eine Herabsetzung der Dosis ist möglich.

Die erfindungsgemäße Verbindung kann intranasal oder parenteral in entsprechender pharmazeutischer Zubereitung verabreicht werden. Für eine intranasale Anwendungsform wird die Verbindung mit den dafür üblichen Zusatzstoffen wie Stabilisatoren oder inerten Verdünnungsmitteln vermischt und durch übliche Methoden in geeignete Darreichungsformen gebracht, wie wäßrige, alkoholische oder ölige Suspensionen oder wäßrige, alkoholische oder ölige Lösungen. Als ölige Trägerstoffe oder Lösungsmittel kommen beispielsweise pflanzliche oder tierische Öle in Betracht, wie Sonnenblumenöl oder Lebertran.

Zur subkutanen oder intravenösen Applikation wird die aktive Verbindung oder deren physiologisch verträgliche Salze, gewünschtenfalls mit den dafür üblichen Substanzen wie Lösungsvermittler, Emulgatoren oder weitere Hilfsstoffe in Lösung, Suspension oder Emulsionen gebracht.

Als Lösungsmittel für die neue aktive Verbindung und die entsprechenden physiologisch verträglichen Salze kommen z. B. in Frage: Wasser, physiologische Kochsalzlösungen oder Alkohole, z. B. Äthanol, Propandiol oder Glycerin, daneben auch Zuckerlösungen, wie Glukose- oder Mannitlösungen, oder auch eine Mischung aus den verschiedenen genannten Lösungsmitteln.

## Beispiel

### 1. Allgemeine Vorschrift zur Peptidkupplung (Tabelle 3)

Zu einer Lösung von 10 mMol einer Z-Aminosäure bzw. eines Z-Peptids und 1,35 g 1-Hydroxybenzotriazol in 10—100 ml Dimethylformamid bzw. Dimethylacetamid gibt man 10 mMol eines Aminosäure- bzw. Peptidester-hydrochlorids, 1,3 ml N-Ethylmorpholin und bei 0°C 2,2 g Dicyclohexylcarbodiimid. Man läßt 2—3 Stunden bei 0°C rühren und anschließend bei Raumtemperatur über Nacht stehen.

### Aufarbeitung I

Der ausgefallene Niederschlag (Dicyclohexylharnstoff) wird abgesaugt und das Filtrat eingeengt. Der Rückstand wird zwischen Wasser und Essigester verteilt. Die Essigesterphase wird nacheinander mit

gesättigter $NaHCO_3$-Lösung, $KHSO_4/K_2SO_4$-Puffer und gesättigter $NaHCO_3$-Lösung ausgeschüttelt, über $Na_2SO_4$ getrocknet und eingeengt. Der Rückstand wird dann meist mit Petrolether verrieben oder aus Essigester/Petrolether umkristallisiert.

Aufarbeitung II

(Die Substanz fällt neben dem Dicyclohexylharnstoff aus): Der Ansatz wird mit 10 ml ges. $NaHCO_3$-Lösung und der etwa 5fachen Wassermenge (bezogen auf Lösungsmittelmenge) verrührt, abgesaugt, mit Wasser gewaschen und über $P_2O_5$ getrocknet.

Tabelle 3

Geschützte Peptide, hergestellt analog allgemeiner Vorschrift 1 und Syntheseschema

| Peptid | Aufarb. Ausb. | Schmp. | $[\alpha]_D^{23}$ (c = l) |
|---|---|---|---|
| Z-Trp-Asn-OBu$^t$ (kristallisiert aus Isopropanol/-Petrolether) | I 76% | 138–39° | −23,1° (in Methanol) |
| Z-Thr(Bu$^t$)-Gly-OEt (kristallisiert aus Petrolether) | I 74% | 76° | +7,2° (in Methanol) |
| Z-Thr(Bu$^t$)-Gly-Trp-Asn-OBu$^t$ (umgefällt aus Essigester/Petroläther) | I 89% | 111° | −19,9° (in Methanol) |
| Z-Ser(Bu$^t$)-Ser(Bu$^t$)-Ser(Bu$^t$)-Thr(Bu$^t$)-Gly-Trp-Asn-OBu$^t$ (mit Methanol ausgekocht) | II 81% | 234° | +7,0° (in Trifluorethanol) |
| Glu-Asp(OBu$^t$)-Ser(Bu$^t$)-Ser(Bu$^t$)-Ser(Bu$^t$)-Thr(Bu$^t$)-Gly-Trp-Asn-OBu$^t$ (Chromatographie an Kieselgel in Methylenchlorid/Methanol wie 9 : 1) | II 38% | 209–214° | +5,1° (in Trifluorethanol) |
| Z-Ser(Bu$^t$)-Ser(Bu$^t$)-OMe | I 85% | Öl | |
| Z-Ser(Bu$^t$)-Ser(Bu$^t$)-Ser(Bu$^t$)-OMe (aus Petrolether) | I 76% | 113–115° | +22,4° (in Methanol) |

2. Allgemeine Vorschrift für die selektive Abspaltung der Benzyloxycarbonylgruppe (Tabelle 4)

2,5–10 g Peptid werden je nach Löslichkeit in etwa 150 ml Methanol oder Trifluoräthanol gelöst oder auch suspendiert. Man gibt unter $N_2$-Spülung Pd/Kohle-Katalysator zu und leitet anschließend unter Rühren und unter Zugabe von ca. 1–2 N methanolischer Salzsäure bei pH 4,5 (Autotitrator) Wasserstoff durch die Lösung. Wenn keine Salzsäure mehr aufgenommen wird, wird wieder mit $N_2$ gespült, filtriert den Katalysator ab und engt das Filtrat ein. Der Rückstand wird in der Regel mit Äther verrieben und abgesaugt.

Tabelle 4

Peptidester-hydrochloride, hergestellt analog allgemeiner Vorschrift 2

| Peptidester-hydrochlorid | Ausb. | Schmp. | $[\alpha]_D^{23}$ (c = l) |
|---|---|---|---|
| H-Trp-Asn-OBu$^t$ · HCl<br>Hydrierung in Methanol | 96% | 108–10° | +7,1°<br>(in Methanol) |
| H-Thr(Bu$^t$)-Gly-Trp-Asn-OBu$^t$ · HCl<br>Hydrierung in Methanol | 93% | 144–46° | –9,2°<br>(in Methanol) |
| H-Ser(Bu$^t$)-Ser(Bu$^t$)-OMe · HCl<br>Hydrierung in Methanol | 98% | Öl | |
| H-Ser(Bu$^t$)-Ser(Bu$^t$)-Ser(Bu$^t$)-Thr(Bu$^t$)-Gly-Trp-Asn-OBu$^t$ · HCl<br>Hydrierung in Trifluorethanol | 93% | 176°<br>(Zers.) | +4,6°<br>(in 80proz. Essigsäure) |

3. Allgemeine Vorschrift für die Verseifung von Peptid-methylestern (Tabelle 5)

10 mMol Peptid werden in 50 ml Dioxan/Wasser wie 8 : 2 gelöst. Dazu gibt man unter Rühren 11 ml 1 N NaOH und läßt eine Stunde bei Raumtemperatur rühren. Danach wird mit wenig (1–2 ml) 1 N $H_2SO_4$ neutralisiert und der Ansatz eingeengt. Der Rückstand wird bei Eiskühlung zwischen 10 ml 1 N $H_2SO_4$ und 50 ml Essigester verteilt. Die Essigesterphase wird nacheinander mit 10 ml $KHSO_4/K_2SO_4$-Puffer und Wasser gewaschen, über $Na_2SO_4$ getrocknet und eingeengt. Aus dem Rückstand lassen sich in Äther die Dicyclohexylamin-Salze herstellen.

Tabelle 5

Z-Peptide, hergestellt analog allgemeiner Vorschrift 3

| Peptid | Ausb. | Schmp. | $[\alpha]_D^{23}$ (c = l) |
|---|---|---|---|
| Z-Thr(Bu$^t$)-Gly-OH | 99% | Öl | |
| Z-Thr(Bu$^t$)-Gly-OH · Dicyclohexylamin | 87% | 142° | +5,6°<br>(in Methanol) |
| Z-Ser(Bu$^t$)-Ser(Bu$^t$)-Ser(Bu$^t$)-OH | 69% | amorph. | +29,5°<br>(in Methanol) |

Glu-Asp-(OBu$^t$)-OH

Zu einer Suspension von 1,9 g (10 mMol) H-Asp(OBu$^t$)-OH und 1,35 g HOBt in 20 ml Dimethylformamid gibt man 3,4 g

Glu-OTcp

und rührt mehrere Stunden bei Raumtemperatur und läßt über Nacht stehen. Anderntags wird im Hochvakuum eingeengt und der Rückstand mit Ether verrieben. Der Niederschlag wird abgesaugt und mit Ether gewaschen. Ausbeute: 2,7 g. Die Substanz enthält noch etwas HOBt und wird deshalb in 70proz. Methanol an Sephadex® LH 20 (Säulenabmessungen: 100 × 4 cm) chromatographiert. Ausbeute: 2,55 g (85%), Schmp. 114–159°, $[\alpha]_D^{23}$ = +1,9° (c = l, Methanol).

Glu-Asp-Ser-Ser-Ser-Thr-Gly-Trp-Asn-OH

850 mg (6,9 mMol)

Glu-Asp($OBu^t$)-Ser($Bu^t$)-Ser($Bu^t$)-Ser($Bu^t$)-Thr($Bu^t$)-Gly-Trp-Asn-$OBu^t$

werden in einer Trifluoressigsäure/Wasser/1.2-Dimercaptoethan-Mischung (wie 9 : 1 : 1) gelöst. Man läßt eine Stunde bei Raumtemperatur stehen, engt ein und verreibt den Rückstand mit Ether. Ausbeute: 650 mg. Zur Reinigung wird die Substanz in 30 ml Methanol aufgekocht und nach dem Abkühlen auf Raumtemperatur abgesaugt. Ausbeute: 430 mg (65%). Aminosäureanalyse (Hydrolyse: 24 Stunden bei 120°C in 6N HCl):

| | Asp | Thr | Ser | Glu | Gly | Trp |
|---|---|---|---|---|---|---|
| Berechnet: | 2 | 1 | 3 | 1 | 1 | 1 |
| Gefunden: | 1,94 | 0,86 | 2,61 | 1,00 | 1,04 | – |

Bei den Hydrolysebedingungen wird Tryptophan völlig, Serin und Threonin teilweise zerstört. Daher erklärt sich die völlige Abwesenheit von Trp und die reduzierten Werte für Ser und Thr. UV-Spektrum: Charakteristische Absorptionsbande für Trp bei 270 nm. Gehalt an Peptid lt. Aminosäureanalyse und UV-Spektrum: 95%. $[\alpha]_D^{23} = -34,9°$ (c = l, in gesättigter $NaHCO_3$-Lösung).

**Patentansprüche für die Vertragsstaaten: BE, CH, DE, FR, GB, IT, LI, LU, NL, SE**

1. Nonapeptid der Formel I

Glu-Asp-Ser-Ser-Ser-Thr-Gly-Trp-Asn-OH (I)

(L-Pyroglutamyl-L-aspartyl-L-seryl-L-seryl-L-seryl-L-threonylglycyl-L-tryptophyl-L-asparagin) und dessen physiologisch verträgliche Salze mit organischen Basen, Alkali- und Erdalkaliionen.

2. Verfahren zur Herstellung einer Verbindung der Formel I, dadurch gekennzeichnet, daß ein geschütztes Peptid der Formel II

Glu-Asp-($OBu^t$)-Ser($Bu^t$)-Ser($Bu^t$)-Ser($Bu^t$)-Thr($Bu^t$)-Gly-Trp-Asn-$OBu^t$ (II)

in der $Bu^t$ für einen tert.-Butylrest oder eine andere Schutzgruppe des tert.-Butyltyps steht, mit in der Peptidchemie bei tryptophanhaltigen Peptiden üblichen Methoden von den Schutzgruppen befreit und das erhaltene Nonapeptid gegebenenfalls in ein physiologisch verträgliches Salz überführt.

3. Verbindung der Formel II.

4. Verbindung der Formel I gemäß Anspruch 1 zur Verwendung als Heilmittel.

5. Heilmittel, enthaltend eine Verbindung der Formel I gemäß Anspruch 1.

**Patentansprüche für den Vertragsstaat: AT**

1. Verfahren zur Herstellung eines Nonapeptids der Formel I

Glu-Asp-Ser-Ser-Ser-Thr-Gly-Trp-Asn-OH (I)

(L-Pyroglutamyl-L-aspartyl-L-seryl-L-seryl-L-seryl-L-threonylglycyl-L-tryptophyl-L-asparagin) und dessen physiologisch verträgliche Salze mit organischen Basen, Alkali- und Erdalkaliionen, dadurch gekennzeichnet, daß ein geschütztes Peptid der Formel II

Glu-Asp-($OBu^t$)-Ser($Bu^t$)-Ser($Bu^t$)-Ser($Bu^t$)-Thr($Bu^t$)-Gly-Trp-Asn-$OBu^t$ (II)

in der $Bu^t$ für einen tert.-Butylrest oder eine andere Schutzgruppe des tert.-Butyltyps steht, mit in der Peptidchemie bei tryptophanhaltigen Peptiden üblichen Methoden von den Schutzgruppen befreit und das erhaltene Nonapeptid gegebenenfalls in ein physiologisch verträgliches Salz überführt.

2. Verfahren zur Herstellung einer Verbindung der Formel II, dadurch gekennzeichnet, daß das Peptid durch Segmentkupplung und anschließende selektive Abspaltung von Schutzgruppen hergestellt wird.

3. Verfahren gemäß Anspruch 1 zur Herstellung einer Verbindung der Formel I zur Verwendung als Heilmittel.

4. Verfahren zur Herstellung eines Heilmittels, enthaltend eine Verbindung der Formel I gemäß Anspruch 1, dadurch gekennzeichnet, daß die Verbindung der Formel I in eine geeignete Darreichungsform gebracht wird.

**Claims for the contracting states: BE, CH, DE, FR, GB, IT, LI, LU, NL, SE**

1. Nonapeptide of the formula I

Glu-Asp-Ser-Ser-Ser-Thr-Gly-Trp-Asn-OH (I)

(L-pyroglutamyl-L-aspartyl-L-seryl-L-seryl-L-seryl-L-threonyl-glycyl-L-tryptophyl-L-asparagine) and its physiologically tolerable salts with organic bases, alkali and alkaline earth metal ions.

2. A process for the preparation of a compound of the formula I as claimed in Claim 1, which comprises liberating a protected peptide of the formula II

Glu-Asp-($OBu^t$)-Ser($Bu^t$)-Ser($Bu^t$)-Ser($Bu^t$)-Thr($Bu^t$)-Gly-Trp-Asn-$OBu^t$ (II)

in which $Bu^t$ is a tert.-butyl radical or another protective group of the tert.-butyl type, from the protective groups according to methods usual in peptide chemistry for tryptophane-containing peptides, and optionally converting the nonapeptide obtained to a physiologically tolerable salt.

3. A compound of the formula II.

4. Compound of the formula I as claimed in Claim 1 for use as medicament.

5. Medicament containing a compound of the formula I as claimed in Claim 1.

**Claims for the contracting state: AT**

1. A process for the preparation of a nonapeptide of the formula I

Glu-Asp-Ser-Ser-Ser-Thr-Gly-Trp-Asn-OH (I)

(L-pyroglutamyl-L-aspartyl-L-seryl-L-seryl-L-seryl-L-threonyl-glycyl-L-tryptophyl-L-asparagine) and its physiologically tolerable salts with organic bases, alkali and alkaline earth metal ions, which comprises liberating a protected peptide of the formula II

Glu-Asp-($OBu^t$)-Ser($Bu^t$)-Ser($Bu^t$)-Ser($Bu^t$)-Thr($Bu^t$)-Gly-Trp-Asn-$OBu^t$ (II)

in which $Bu^t$ is a tert.-butyl radical or another protective group of the tert.-butyl type, from the protective groups according to methods usual in peptide chemistry for tryptophane-containing peptides, and optionally converting the nonapeptide obtained to a physiologically tolerable salt.

2. A process for the preparation of a compound of the formula II, which comprises preparing the peptide by segment coupling followed by selectively splitting off the protective groups.

3. A process as claimed in Claim 1 for the preparation of a compound of the formula I for use as medicament.

4. A process for the preparation of a medicament containing a compound of the formula I as claimed in Claim 1, which comprises converting the compound of the formula I to a suitable form of administration.

**Revendications pour les Etats contractants: BE, CH, DE, FR, GB, IT, LI, LU, NL, SE**

1. Nonapeptide de formule I

Glu-Asp-Ser-Ser-Ser-Thr-Gly-Trp-Asn-OH (I)

(L-pyroglutamyl-L-aspartyl-L-séryl-L-séryl-L-séryl-L-thréonylglycyl-L-tryptophyl-L-asparagine) et ses sels physiologiquement acceptables avec des bases organiques, des ions alcalins et alcalinoterreux.

2. Procédé de préparation d'un composé de formule I selon la revendication 1, caractérisé en ce que, par des procédés usuels dans la chimie des peptides pour des peptides contenant du tryptophane, on

libère des groupes protecteurs un peptide protégé de formule II

Glu-Asp-($OBu^t$)-Ser($Bu^t$)-Ser($Bu^t$)-Ser($Bu^t$)-Thr($Bu^t$)-Gly-Trp-Asn-$OBu^t$ (II)

dans laquelle $Bu^t$ représente un groupe butyle tertiaire ou un autre groupe protecteur du type butyle tertiaire et on convertit éventuellement le nonapeptide obtenu en un sel physiologiquement acceptable.

3. Composé de formule II.

4. Composé de formule I selon la revendication 1, pour utilisation comme médicament.

5. Médicament contenant un composé de formule I selon la revendication 1.

**Revendications pour l'Etat contractant: AT**

1. Procédé de préparation d'un nonapeptide de formule I

Glu-Asp-Ser-Ser-Ser-Thr-Gly-Trp-Asn-OH (I)

(L-pyroglutamyl-L-aspartyl-L-séryl-L-séryl-L-séryl-L-thréonylglycyl-L-tryptophyl-L-asparagine) et ses sels physiologiquement acceptables avec des bases organiques, des ions alcalins et alcalinoterreux, caractérisé en ce que, par des procédés usuels dans la chimie des peptides pour des peptides contenant du tryptophane, on libère des groupes protecteurs un peptide protégé de formule II

Glu-Asp-($OBu^t$)-Ser($Bu^t$)-Ser($Bu^t$)-Ser($Bu^t$)-Thr($Bu^t$)-Gly-Trp-Asn-$OBu^t$ (II)

dans laquelle $Bu^t$ représente un groupe butyle tertiaire ou un autre groupe protecteur du type butyle tertiaire, et on convertit éventuellement le nonapeptide en un sel physiologiquement acceptable.

2. Procédé de préparation d'un composé de formule II, caractérisé en ce qu'on prépare le peptide par couplage de segments, puis par élimination sélective des groupes protecteurs.

3. Procédé selon la revendication 1 pour la préparation d'un composé de formule I, utilisable comme médicament.

4. Procédé de préparation d'un médicament contenant un composé de formule I selon la revendication 1, caractérisé en ce que le composé de formule I est mis sous une forme d'administration appropriée.